# EUROPEAN PATENT SPECIFICATION

(11) **EP 4 104 847 B1**
(45) Date of publication and mention of the grant of the patent: **03.09.2025**
(21) Application number: 22178442.4
(22) Date of filing: 10.06.2022
(51) Int. Cl.: A61K 36/88, A61K 35/747, A61K 35/745, A61K 35/744, A61K 35/00, A61P 1/10, A61P 25/24

(54) **CROCUS SATIVUS AND PROBIOTICS FOR THE TREATMENT OF DEPRESSIVE DISORDER AND COPROSTASIS**
CROCUS SATIVUS UND PROBIOTIKA ZUR BEHANDLUNG VON DEPRESSIVEN STÖRUNGEN UND KOPROSTASE
CROCUS SATIVUS ET PROBIOTIQUES POUR LE TRAITEMENT DES TROUBLES DÉPRESSIFS ET DE LA COPROSTASE

(30) Priority: 16.06.2021 EP 21179799
(43) Date of publication of application: 21.12.2022
(73) Proprietor: Galenus G.H. AG, 6052 Hergiswil (CH)
(72) Inventor: Dobmeyer, Rita, 6052 Hergisvil (CH); Naber, Kurt, 94315 Straubing (DE)
(74) Representative: Kompter, Hans-Michael

(56) References cited:
- WO-A1-2016/065419
- CN-A- 112 334 145
- FR-A1- 3 004 110
- GR-B- 1 010 023
- US-A1- 2023 201 297
- ADRIAN L LOPRESTI ET AL: "Saffron (Crocus sativus) for depression: a systematic review of clinical studies and examination of underlying antidepressant mechanisms of action", HUMAN PSYCHOPHARMACOLOGY. CLINICAL AND EXPERIMENTAL, JOHN WILEY & SONS LTD, XX, vol. 29, no. 6, 22 September 2014 (2014-09-22), pages 517 - 527, XP071721084, ISSN: 0885-6222, DOI: 10.1002/HUP.2434
- BACTOFLOR: "BactoFlor 10/20 - BactoFlor | Bakterienkulturen in Premiumqualität", 21 March 2019 (2019-03-21), XP093095394, Retrieved from the Internet <URL:https://bactoflor.de/bactoflor-10-20> [retrieved on 20231026]

## Description

### BACKGROUND OF THE INVENTION

### 1. TECHNICAL FIELD

The present invention relates to a composition for the treatment of a patient suffering from a depressive disorder and coprostatis, wherein the composition comprises as the active ingredients a combination of therapeutically effective amounts of an extract of saffron stigmas (*Crocus sativus L*.) (1) and one or more non-pathogenic probiotic microorganisms (2).

### 2. PRIOR ART

This invention falls within the field of the pharmaceutical industry, specifically within the field of the treatment or prevention of mood disorders, in particular related to depression.

After hypertension, depressive disorder is the most common medical condition in developed countries (Akhondzadeh et al., 2008). The World Health Organization (WHO) defines it as the most common of mental disorders, which affects a high percentage of the population. The most common symptoms are weight loss, anxiety, hypochondria, insomnia, somatic and sexual disorders, feelings of guilt, suicide, etc. (Hamilton, 1960). Long periods of depression can lead to the onset of chronic diseases such as heart disease, metabolic diseases such as diabetes or hormonal disorders, among others (Moussavi et al., 2007, Bisschop et al., 2004).

Given that one of the main causes of the onset of depression is the decrease of some neurotransmitters related to mood (Carr et al., 2011, Lopresti and Drummond, 2014), the most commonly used antidepressants are inhibitors. selective reuptake of these neurotransmitters (Serretti et al., 2007). Among the main neurotransmitters responsible for mood are serotonin, norepinephrine (noradrenaline) and dopamine. One of the main disadvantages of synthetic drug treatments is the appearance of side effects, which can range from mild, such as dry mouth, headache, nausea, diarrhea, etc., to severe, such as tachycardia, sexual dysfunction, hypertension, hypercholesterolemia ... etc. (Ferguson, 2001; Vanderkooy, 2002).

Due to the high security requirements of antidepressant treatments and the associated side effects, in recent years, research has focused on plant extracts with possible psychopharmacological application. These include saffron-based products {*Crocus safivus L*), the effect of which has been evidenced scientifically with both *in vivo* studies in experimental animals and clinical studies with people with pathologies related to depression (Akhondzadeh, 2004 , 2005, 2008, Bastí et al., 2007, Noorbala et al., 2005, Moshiri et al., 2006, Ulbricht et al., 201 1, Kashani, 2013, Shahmansouri et al., 2014 Siddiqui et al., 2018). These investigations have shown that the safranal and the crocids present in the saffron extracts studied are responsible for the increase in the concentration of neurotransmitters in brain tissue (Karimi et al., 2001, Hosseinzadeh et al., 2004, Noorbala et al. al., 2005; Ettehadi et al., 2013).

The International patent application WO2004/098622 suggests administering probiotics in the treatment of atypical depression.

The French patent application FR 3 004 110 A1 discloses a nutritional or pharmaceutical active ingredient for oral administration comprising the combination of an extract of Crocus sativus, and at least one probiotic bacterial strain for or preventing and/or controlling anxiety, anger, social hostility, obsessive-compulsive disorders, paranoid ideas and/or mild depression.

The Greek patent GR 1 010 023 B teaches that the triple combination of probiotics, magnesium and Crocus Sativus L extract exhibits enhanced activity relative to the corresponding combination of probiotics and Crocus Sativus L. extract in combating stress, anger, social anxiety, obsessive-compulsive disorder, paranoid thoughts and / or mild depression.

There are several products on the market based on saffron, however, they are not efficient enough to treat mood disorders in a way, to relief the patients completely from their depressive disorder and coprostatis.

Therefore, in view of the above, there is a need to provide a composition based on a natural extract of saffron that effectively treats or prevents depressive disorder and coprostatis but without the negative side effects associated with the use of synthetical drugs.

Surprisingly, it has been found that this problem can be solved by a combination of a natural extract of saffron with probiotic microorganisms.

### BRIEF DESCRIPTION OF THE INVENTION

The present invention relates to a composition for use in the method for the treatment and/or prevention of a depressive disorder and coprostatis, comprising as the active ingredients a combination of therapeutically effective amounts of an extract of saffron stigmas (*Crocus sativus L*.) (1) and non-pathogenic probiotic microorganisms (2) and an acceptable excipient, wherein the ratio of (1) to (2) ranges from 100 to 1 to 1 to 100 by weight, wherein (2) comprises *Bifidobakterium bifidum, Bifidobakterium breve, Bifidobakterium longum, Lactobacillus acidophilus, Lactobacillus casei, Lactobacillus reuteri, Lactobacillus rhamnosus, Lactobacillus plantarum, Lactococcus lactis, and Enterococcus faecium.*

### DETAILED DESCRIPTION OF THE INVENTION

To obtain the saffron extract of the invention, a strict selection of the raw material is first made, based mainly on the determination of safranal active ingredients and crocins by HPLC, microbial load, etc. The extraction of the active principles of the selected saffron stigmas is preferably carried out with water or hydroalcoholic mixtures as long as the proportion of ethanol does not exceed 20% (v / v). Temperature control during the extraction process is critical and should be limited to 70 ° C to avoid deterioration of some isomers of crocuses that may be affected under higher temperature conditions.

Organic solvents such as ethyl acetate, hexane, petroleum ether, acetone, methanol or the like are not allowed to obtain the saffron extract to be used according to the present invention.

The crude extract is in the form of a dense liquid, with a deep red color and intense aroma characteristic of saffron, which is cooled to room temperature with subsequent elimination of humidity. Different drying techniques can be used for this stage, provided that the temperature remains below 70 ° C.

The last step consists of an adaptation of the particle size by grinding the dry extract in a hammer mill or blades, always checking that the grinding temperature does not affect the bioactive compounds, and a sieving of the grind through a light sieve of maximum mesh of 240 pm. The crude extract is mixed with the one or more probiotic microorganisms and a pharmaceutically acceptable excipient to prepare the composition of the present invention.

The saffron extract comprises as active ingredients safranal (2,6,6-Trimethylcyclohexa-1,3-diene-1-carbaldehyde), crocin (bis[(2S,3R,4S,5S,6R)-3,4,5-trihydroxy-6-({[(2R,3R,4S,5S,6R)-3,4,5-trihydroxy-6-(hydroxymethyl)oxan-2-yl]oxy}methyl)oxan-2-yl] (2E,4E,6E,8E,10E,12E,14E)-2,6,11,15-tetramethylhexadeca-2,4,6,8,10,12,14-heptaenedicate) und picrocrocin (4-(β-D-glucopyranosyloxy)-2,6,6-trimethyl-1-cyclohexene-1-carbaldehyde).

In another embodiment of the present invention the depressive disorder is selected from the group consisting of unipolar depression, bipolar depression, preferably treatment-resistant depression, in particular resistant against treatment with St. Johns Wort (*Hypericum perforatum*), or mild to moderate depression, in particular post-partum mild to moderate depression..

In another embodiment of the present invention, the composition as described above, further comprises at least one pharmacologically acceptable excipient.

The amounts of individual microbial strains to be administered to subjects or to be included in compositions disclosed herein will depend on a variety of factors including the identity and number of individual strains employed, the nature and extent of any condition suffered by the subject, and the form in which a composition is administered. For any given case, appropriate amounts may be determined by one of ordinary skill in the art using only routine experimentation.

By way of example only, the amount of each microbial strain present in a daily dose of a composition disclosed herein may be from about 1 x 10² colony forming units (cfu) to about 1 x 10¹¹ cfu, and may be about 1 x 10³ cfu, about 2.5 x 10³ cfu, about 5 x 10³ cfu, about 7.5 x 10³ cfu, 1 x 10⁴ cfu, about 2.5 x 10⁴ cfu, about 5 x 10⁴ cfu, about 7.5x 10⁴ cfu, about 1 x 10⁵ cfu, about 2.5 x 10⁵ cfu, about 5 x 10⁵ cfu, about 7.5 x 10⁵ cfu, about 1 x 10⁶ cfu, about 2.5 x 10⁶ cfu, about 5 x 10⁶ cfu, about 7.5 x 10⁶ cfu, about 1 x 10⁷ cfu, about 2.5 x 10⁷ cfu, about 5 x 10⁷ cfu, about 7.5 x 10⁷ cfu, about 1 x 10⁸ cfu, about 2.5 x 10⁸ cfu, about 5 x 10⁸ cfu, about 7.5 x 10⁸ cfu, about 1 x 10⁹ cfu, about 2.5 x 10⁹ cfu, about 5 x 10⁹ cfu, about 7.5 x 10⁹ cfu, about 1 x 10¹⁰ cfu, about 2.0 x 10¹⁰ cfu, about 5 x 10¹⁰ cfu, about 7.5x 10¹⁰ cfu, and about 1 x 10¹¹ cfu.

By way of example only, the amount of each microbial strain present in a single dosage form of a composition disclosed herein (e.g. per capsule) may be from about 1 x 10 cfu to about 1 x 10¹¹ cfu, and may be about 1 x 10³ cfu, about 2.5 x 10³ cfu, about 5 x 10³ cfu, about 7.5 x 10³ cfu, 1 x 10⁴ cfu, about 2.5 x 10⁴ cfu, about 5 x 10⁴ cfu, about 7.5x 10⁴ cfu, about 1 x 10⁵ cfu, about 2.5 x 10⁵ cfu, about 5 x 10⁵ cfu, about 7.5 x 10⁵ cfu, about 1 x 10⁶ cfu, about 2.5 x 10⁶ cfu, about 5 x 10⁶ cfu, about 7.5 x 10⁶ cfu, about 1 x 10⁷ cfu, about 2.5 x 10⁷ cfu, about 5 x 10⁷ cfu, about 7.5 x 10⁷ cfu, about 1 x 10⁸ cfu, about 2.5 x 10⁸ cfu, about 5 x 10⁸ cfu, about 7.5 x 10⁸ cfu, about 1 x 10⁹ cfu, about 2.5 x 10⁹ cfu, about 5 x 10⁹ cfu, about 7.5 x 10⁹ cfu, about 1 x 10¹⁰ cfu, about 2.0 x 10¹⁰ cfu, about 5 x 10¹⁰ cfu, about 7.5x 10¹⁰ cfu, and about 1 x 10¹¹ cfu.

By way of example only, the combined amount of probiotic microbial strains present in a daily dose of a composition disclosed herein may be from about 1 x 10 cfu to about 1 x 10¹¹ cfu, and may be about 1 x 10³ cfu, about 2.5 x 10³ cfu, about 5 x 10³ cfu, about 7.5 x 10³ cfu, 1 x 10⁴ cfu, about 2.5 x 10⁴ cfu, about 5 x 10⁴ cfu, about 7.5x 10⁴ cfu, about 1 x 10⁵ cfu, about 2.5 x 10⁵ cfu, about 5 x 10⁵ cfu, about 7.5 x 10⁵ cfu, about 1 x 10⁶ cfu, about 2.5 x 10⁶ cfu, about 5 x 10⁶ cfu, about 7.5 x 10⁶ cfu, about 1 x 10⁷ cfu, about 2.5 x 10⁷ cfu, about 5 x 10⁷ cfu, about 7.5 x 10⁷ cfu, about 1 x 10⁸ cfu, about 2.5 x 10⁸ cfu, about 5 x 10⁸ cfu, about 7.5 x 10⁸ cfu, about 1 x 10⁹ cfu, about 2.5 x 10⁹ cfu, about 5 x 10⁹ cfu, about 7.5 x 10⁹ cfu, about 1 x 10¹⁰ cfu, about 2.0 x 10¹⁰ cfu, about 5 x 10¹⁰ cfu, about 7.5x 10¹⁰ cfu, and about 1 x 10¹¹ cfu.

By way of example only, the combined amount of probiotic microbial strains present in a single dosage form of a composition disclosed herein (e.g. per capsule) may be from about 1 x 10² cfu to about 1 x 10¹¹ cfu, and may be about 1 x 10³ cfu, about 2.5 x 10³ cfu, about 5 x 10³ cfu, about 7.5 x 10° cfu, 1 x 10⁴ cfu, about 2.5 x 10⁴ cfu, about 5 x 10⁴ cfu, about 7.5x 10⁴ cfu, about 1 x 10⁵ cfu, about 2.5 x 10⁵ cfu, about 5 x 10⁵ cfu, about 7.5 x 10⁵ cfu, about 1 x 10⁶ cfu, about 2.5 x 10⁶ cfu, about 5 x 10⁶ cfu, about 7.5 x 10⁶ cfu, about 1 x 10⁷ cfu, about 2.5 x 10⁷ cfu, about 5 x 10⁷ cfu, about 7.5 x 10⁷ cfu, about 1 x 10⁸ cfu, about 2.5 x 10⁸ cfu, about 5 x 10⁸ cfu, about 7.5 x 10⁸ cfu, about 1 x 10⁹ cfu, about 2.5 x 10⁹ cfu, about 5 x 10⁹ cfu, about 7.5 x 10⁹ cfu, about 1 x 10¹⁰ cfu, about 2.0 x 10¹⁰ cfu, about 5 x 10¹⁰ cfu, about 7.5x 10¹⁰ cfu, and about 1 x 10¹¹ cfu, preferably about 1 x 10¹⁰ cfu, to about 5 x 10¹⁰ cfu, in particular 2.0 x 10¹⁰ cfu.

The bacterial strains to be employed in accordance with the present disclosure may be cultured according to any suitable method known to the skilled addressee and may be prepared for addition to a composition by, for example, freeze-drying, spray-drying or lyophilisation. Thus, in embodiments of the present disclosure the bacterial strains may be in a dried form (such as lyophilized or sporulated form) in a suitable carrier medium, for example a FOS medium or other soluble fibre, sugar, nutrient or base material for the composition, with which the bacterial strains can be presented in an orally administrable form. One or more of the strains may be microencapsulated in, for example, a suitable polymeric or protein matrix to improve long term stability and storage of the compositions. In one example, microencapsulation may comprise alginate beads, although those skilled in the art will appreciate that any suitable microencapsulation material or matrix may be used. Microencapsulation may be achieved using methods and techniques known to those skilled in the art.

As used herein, the term "acceptable" relates to molecular entities and compositions that are physiologically tolerable and do not normally cause an allergic reaction or a similar adverse reaction, such as gastric discomfort, dizziness and the like, when administered to humans. As used herein, the term "acceptable" preferably means pharmaceutically acceptable, acceptable for medicinal products or devices or, nutraceutically acceptable. Furthermore, pharmaceutically acceptable, means that it is approved by a regulatory agency of the federal or state government or listed in the US pharmacopoeia or another pharmacopoeia, generally recognized for its use in animals, preferably in mammals and more particularly in human beings.

Pharmaceutical compositions are preparations made from substances that are used to heal or alleviate illnesses or to ensure that illnesses or complaints do not occur in the first place. This applies to both human and animal use. The substances can act in the body or on the body.

Neutraceutical compositions or dietary supplements are foods. Therefore, on the one hand, they are subject to the extensive legal provisions that apply to all foods. On the other hand, there are additional, special regulations for food supplements, e.g., regarding to composition and labeling. At the European level, these requirements are primarily contained in Directive 2002/46 / EC on food supplements.

Neutraceuticals are intended to supplement the general diet, they as a rule consist of natural products, e.g., vitamins or minerals or other substances with a nutritional or physiological effect and are in concentrated form, are put on the market in dosed form for absorption in measured small quantities.

As used herein, the term "in combination with" or "co-administered" covers both separate and sequential administration of the active agents. For example, when the agents are administered sequentially, either the extract of saffron or the one or more probiotic microorganisms may be administered first. When administration is simultaneous, the agents may be administered either in the same or a different pharmaceutical composition. Adjunctive therapy, i.e., where one agent is used as a primary treatment and the other agent is used to assist that primary treatment, is also an embodiment of the present invention.

Accordingly, a composition for the claimed use is preferred, wherein the composition is prepared from a ready-to-use kit of parts essentially consisting of
(A) a first compartment containing a pharmaceutical composition comprising effective amounts of an extract of saffron stigmas (*Crocus sativus L*.) (1) and an acceptable excipient;
(B) a second compartment containing a pharmaceutical composition comprising non-pathogenic probiotic microorganisms (2) and an acceptable excipient;
(C) optionally a leaflet describing the dosage and administration of each of the pharmaceutical compositions (A) and (B),
wherein
the ratio of (1) to (2) ranges from 100 to 1 to 1 to 100 by weight, and

(2) comprises *Bifidobakterium bifidum, Bifidobakterium breve, Bifidobakterium longum, Lactobacillus acidophilus, Lactobacillus casei, Lactobacillus reuteri, Lactobacillus rhamnosus, Lactobacillus plantarum, Lactococcus lactis,* and *Enterococcus faecium.*

By "effective" amount of an active principle, a drug, formulation, or permeant is meant a sufficient amount of the active principle to provide the effect. A "topically effective," "cosmetically effective," "pharmaceutically effective", dietary effective", medicinal effective", "preventively effective", or "therapeutically effective" amount refers to the amount of an active ingredient needed to effect the desired effect.

The term "synergistically effective" amount of a combination of active ingredients means that the effect of the combination of the active principles is higher than the sum of the expected effects of both the active ingredients, when being administered alone. In a preferred embodiment, the extract of saffron stigmas and the one or more probiotic microorganisms are administered in synergistically effective amounts.

In a preferred embodiment, the composition according to the invention comprises an extract of saffron stigmas (1) and non-pathogenic probiotic microorganisms (2), wherein the ratio of (1) to (2) ranges from 20 to 1 to 1 to 20 by weight.

Preferably, the composition according to the invention comprises
- 10 to 150 mg, preferably 20 to 100 mg, in particular 25 to 90 mg, most preferred about 30, about 60 or about 88.5 mg of an extract of saffron stigmas (1), and
- 1 x 10 cfu to 1 x 10¹¹ cfu, preferably about 1 x 10¹⁰ cfu, to about 5 x 10¹⁰ cfu, in particular about 2.0 x 10¹⁰ cfu of non-pathogenic probiotic microorganisms (2),
wherein the ratio of (1) to (2) ranges from 100 to 1 to 1 to 100 by weight.

Another preferred embodiment is a composition according to the invention, wherein the effective amounts
- of an extract of saffron stigmas (1), which is from 10 mg to 150 mg, preferably 20 to 100 mg, in particular 25 to 90 mg, most preferred about 30, about 60 or about 88.5 mg, and
- of non-pathogenic probiotic microorganisms (2), which is from 1 x 10 cfu to 1 x 10¹¹ cfu, preferably 1 x 10¹⁰ cfu, to 5 x 10¹⁰ cfu, in particular about 2.0 x 10¹⁰ cfu,
are administered once daily.

The term "excipient" refers to a substance that aids the absorption of any of the components of the product of the invention, stabilizes said components or assists in the preparation of the pharmaceutical composition. Thus, the excipients can have the function of keeping the components together, such as, for example, starches, sugars or celluloses; sweetening, coloring, protective function of the drug from the external medium, such as for example to isolate it from air and / or humidity; filling function of a tablet, capsule or any other form of presentation, such as dibasic calcium phosphate; disintegrating function to facilitate the dissolution of the components and their absorption in the intestine, without excluding other types of excipients not mentioned in this paragraph. Therefore, the term "excipient" is defined as that material, included in galenic forms, is added to the active ingredients or their associations to enable their preparation and stability, modify their organoleptic properties or determine the physico-chemical properties of the pharmaceutical composition and its bioavailability. The "pharmaceutically acceptable" excipient must not interact with the activity of the active compounds of the pharmaceutical composition. Examples of excipients are binders, fillers, disintegrators, lubricants, coaters, sweeteners, flavorings and colorants. More specific non-limiting examples of acceptable excipients are starches, sugars, xylitol, sorbitol, calcium phosphate, spheroidal fats, talc, silica or glycerin, among others.

In a preferred embodiment of the present invention, the composition is a pharmaceutical composition, and comprises pharmaceutically acceptable excipients. The pharmaceutical composition is the formulation of the set of components that form at least the product of the invention (the composition comprising a saffron extract), which has at least one application in improving the physical, physiological and / or psychological state of a subject, which implies a general improvement of their health status, as well as an increase in their quality of life.

In the present invention the term "subject" is equivalent to the term "individual", so both terms can be used interchangeably in the present invention. "Subject" means any animal belonging to any species. Examples of subjects include, but are not limited to, animals of commercial interest such as birds (chickens, ostriches, chickens, geese, partridges, etc.), rabbits, hares, domestic or companion animals (dogs, cats, etc.), sheep (sheep , etc.), goats (goats, etc.), swine (pigs, pigs, etc.), equine livestock (horses, ponies, etc.) and cattle or cattle (bulls, oxen, etc.). In a particular embodiment, the subject is a mammal, preferably a primate, more preferably a human being of any race, sex, or age. Since all the components of the pharmaceutical composition of the invention are compatible for elderly people, it can be used for the treatment of geriatric patients. The term "geriatric patients" as used hereinbefore and hereinbelow includes senior people of an age of 60 years onwards, who may be or may suffer from depressive disorders and coprostatis.

"Disorder" is understood as the behavioral or psychological pattern of clinical significance that, whatever its cause, is an individual manifestation of a psychological or biological dysfunction. This manifestation is considered a symptom when it appears associated with discomfort (for example, pain), a disability (for example, deterioration in an area of functioning) or a significantly increased risk of dying or suffering pain, disability or loss of consciousness. The International Classification of Diseases or ICD 10 classifies these disorders under the section called "Mood Disorders (Affective)".

Two groups of mood disorders are usually differentiated, depending on whether or not they include the presence of episodes of mania or hypomania: depressive disorders and bipolar disorders. In the context of the present invention, the composition, as described above, is directed to the prevention of depressive disorders. Examples of depressive disorders include, but are not limited to, atypical depression, melancholic depression, psychotic depression, catatonic depression, postpartum depression, seasonal affective disorder, dysthymia, double depression, depressive disorder not otherwise specified, depressive disorder of the personality, recurrent brief depressive disorder, and minor depression.

The efficacy of the composition according to the invention can be evaluated according to the Center for Epidemiologic Studies Depression Scale (CES-D), NIMH. The CES-D scale is a brief self-report scale designed to measure self-reported symptoms associated with depression experienced in the past week. The items of the scale are symptoms associated with depression, which have been used in previously validated longer scales.

The CES-D scale has been shown to be a reliable measure for assessing the number, types, and duration of depressive symptoms across racial, gender, and age categories Radloff, 1977). High internal consistency has been reported with Cronbach's alpha coefficients ranging from .85 to .90 across studies (Radloff, 1977). Concurrent validity by clinical and self-report criteria, as well as substantial evidence of construct validity have been demonstrated (Radloff, 1977). The CES-D scale runs from 0 (no depression) to 60 points (extremely depressed). A score between 16 and 28 points is considered mild to moderate depression.

Preferably, the treatment with the composition according to the invention will reduce the depressive disorder of the patient in need thereof by at least 20 %, preferably by 25 to 60 %, in particular by35 to 50 according to the CES-D scale.

In an embodiment of present invention, the composition, as described above, is administered orally.

In another embodiment of the present invention, the composition, as described above, is administered in a dose greater than 25 mg / day, preferably the daily dose is between 40 and 250 mg / day, more preferably the dose daily is between 60 and 200 mg / day, still more preferably the daily dose is between 75 and 175 mg / day, even more preferably the daily dose is about 140 mg / day. For its therapeutic application, the product of the invention will be in a pharmaceutically acceptable or substantially pure form, that is, having a pharmaceutically acceptable degree of purity excluding the usual pharmaceutical additives as diluents and carriers, and not including material considered toxic at levels of normal dosage.

The purity grades of the saffron extract of the present invention are greater than 50%, preferably greater than 70%, and still more preferably greater than 90%. The "galenical form or pharmaceutical form" is the provision to which the active ingredients and excipients are adapted to constitute a medicine. It is defined by the combination of the way in which the pharmaceutical composition is presented by the manufacturer and the way in which it is administered.

The pharmaceutical composition may comprise a "carrier" or carrier, which is preferably an inert substance. The function of the vehicle is to facilitate the incorporation of other compounds, to allow a better dosage and administration or to give consistency and form to the pharmaceutical composition. Thus, the vehicle is a substance that is used to dilute any of the components of the pharmaceutical composition of the present invention to a certain concentration; or that even without diluting said components is able to allow a better dosage and administration or give consistency and form to the medicine. Therefore, it would be considered a pharmaceutically acceptable vehicle. When the form of presentation of the extract is liquid, the pharmaceutically acceptable carrier is the diluent. In addition, the excipient and the vehicle must be pharmacologically acceptable.

Non-limiting examples of excipients are starch, lactose, dextrose, maltodextrin, sucrose, mannitol, sorbitol, glucose, microcrystalline cellulose, di- and tricalcium phosphate, calcium sulfate, magnesium stearate, silica, kaolin and sodium chloride. Preferably, the excipient is obtained from dextrin, maltodextrin or any of its mixtures.

The pharmaceutical composition of the invention may comprise other active substances. In addition to the requirement of therapeutic efficacy, where said pharmaceutical composition may necessitate the use of other therapeutic agents, there may be additional fundamental reasons that require or strongly recommend the use of a combination of a compound of the invention and another therapeutic agent. The term "active ingredient" is any material, whatever its origin, human, animal, plant, chemical or otherwise, to which a specific activity is attributed to constitute a medicine. In a preferred embodiment the composition of the invention further comprises a salt or complex of a metal, which is effective against mood disorders selected from the group consisting of lithium and zinc.

In each case, the form of presentation of the medication will be adapted to the route of administration chosen. Therefore, the composition of the present invention can be presented in the form of solutions or any other form of clinically permitted administration and in an effective therapeutic dose. The pharmaceutical composition of the invention can be presented in solid, semi-solid, liquid or gaseous form. As a tablet, capsule, powder, granule, ointment, solution, suppository, injectable, inhalant, gel, syrup, nebulizer, microsphere or aerosol. Preferably the composition is adapted for oral administration, such as, for example, in the form of a tablet, capsule, powder, granule, solution or syrup, in particular a tablet, hard capsule or soft-shell capsule.

In another embodiment of the present invention, the composition, as described above, is administered as a tablet, capsule, powder, granule or solution. Most preferred are gelatin capsules comprising as additional carrier cellulose, magnesium stearate and/or silica.

The compositions of the present invention can be prepared using conventional methods, such as those described in the Pharmacopoeias of different countries and in other reference texts. The compounds and compositions of the present invention can be administered together with other medicaments in combination therapies.

In addition to what has been described in previous paragraphs, the possibility that the composition, as described above, can be administered to a subject jointly with other compounds, whether they are part of or not, is also contemplated in the present invention.

Disclosed herein but not claimed is the the use of the composition, as described above, as a food supplement. By food supplement is understood the products marketed in the form of capsules, tablets, ampoules, tisanes, drinking solutions, etc., whose purpose is to complete the usual diet and which are a concentrated source of nutrients (such as vitamins, minerals, amino acids, essential fatty acids, fibers, etc.) or other substances that have a nutritional or physiological Disclosed herein but not claimed is a functional food comprising the composition, as described above. A functional food is that food that, beyond satisfying the nutritional needs, can produce a specific beneficial effect for health associated with the prevention or reduction of the risk of contracting specific pathologies. This beneficial effect is achieved mainly by the addition, modification or elimination of certain components present in food.

Throughout the description and claims the word "comprises" and its variants do not intend to exclude other technical characteristics, additives, components or steps. For those skilled in the art, other objects, advantages and characteristics of the invention will emerge partly from the description and partly from the practice of the invention. The following examples are provided by way of illustration, and are not intended to be limiting of the present invention.

### EXAMPLES

The invention will now be illustrated by means of tests carried out by the inventors, which highlights the composition and activity of the composition of the invention.

### Example 1 - Case reports

### Case A

A female patient of 69 years suffered from coprostasis for more than 15 years and mild to moderate depression following the CES-D scale for the same time including symptoms like fatigue, sleeplessness, restlessness and the feel of not make it to "get going"

Treatment started with 900 mg of extract of St. John's Wort (*Hypericum perforatum*) daily in 3 divided doses for up to 12 weeks (range, 200 to 1,800 mg/day), which did not change patient's status for 3 months.

After 4 weeks wash out the patent is given an extract of saffron stigmas (*C. sativus L*). at a daily dose of 30 mg/day.

After further 4 weeks the patient feels 15 % better according to the CES-D scale. However, sleeplessness, fatigue and coprostatis still remain.

Subsequently, the patient is given once daily an additional medication (1 capsule, 110 mg) consisting of 10 mg of inulin and 2.0 x 10¹⁰ cfu of the following microencapsulated probiotic microorganisms:
*Bifidobakterium bifidum, Bifidobakterium breve, Bifidobakterium longum, Lactobacillus acidophilus, Lactobacillus case, Lactobacillus reuteri, Lactobacillus rhamnosus, Lactobacillus plantarum, Lactococcus lactis,* and *Enterococcus faecium*

This probiotic composition is commercially available under the tradename Bactoflor^{®} 10/20 which is commercially available from Intercell Pharma GmbH.

After only 1 week the patient is able to defecate spontaneously and almost daily. Sleep pattern is significantly better and overall improvement by 40 % is noticed on the CES-D scale.

### Case B

A male patient of 62 years suffered from mild to moderate depression following the CES-D scale including symptoms like fatigue, sleeplessness, restlessness, and anxiety.

Treatment started with St. John's Wort (*Hypericum perforatum* 900 mg of extract daily in 3 divided doses for up to 12 weeks (range, 200 to 1,800 mg/day) did not change patient's status for 3 months.

After 4 weeks wash out the patent is given once daily 110 mg of the probiotic Bactoflor^{®} 10/20 (cp. Case A)

After further 4 weeks the patient feels 10 % better according to the CES-D scale. However, sleeplessness, fatigue and anxiety remain.

Subsequently, the patient is given an extract of petal of *C. sativus* L. at a daily dose of 30 mg/day as an additional medication.

After 2 weeks the sleep pattern is significantly better and overall improvement by 43 % is noticed on the CES-D scale.

### CONCLUSION

The comparison of the results obtained in the case reports A and B clearly proves that the combined administration of an extract of saffron stigmas and a probiotic composition containing several Bifidobacterium species and several Lactobacillus species shows a synergistic effect against mild to moderate depression.

### REFERENCES

Akhondzadeh, S., Fallah-Pour, H., Afkham, K., Jamshidi, AH. , Khalighi-Cigaroudi, F. (2004). BMC Complementary and Alternative Medicine 4, 12.
Akhondzadeh, S., Tahmacebi-Pour, N., Noorbala, AA., Amini, H., Fallah-Pour, H., Jamshidi, AH. , Khani, M. (2005). Phytotherapy Research, 19, 148-151.
Akhondzadeh BA. , Ghoreishi SA., Noorbala AA., Akhondzadeh, SH., Rezazadeh SH. (2008). Journal of Medicinal Plants, 7, 29-36.
Bastí, AA., Moshiri, E., Noorbala, AA., Jamshidi, AH., Abbasi, SH., Akhondzadeh, S. (2007), Progress in Neuro- Psychopharmacology & Biological Psychiatry, 31, 439-442.
Bisschop, MI , Kriegsmana, DMW. , Beekman, ATF. , Deeg, DJH. (2004). Social Science & Medicine, 59, 721-733.
Buysse, DJ., Reynolds, CF. , Monk, TH. , Berman, SR., Kupfer, DJ. (1989). Psychiatry Research 28, 193-213.
Carr, GV., Schechter, LE., Lucki, I. (2011). Psychopharmacology, 213, 499-507.
Ettehadi, H., Mojabi, SN. , Ranjbaran, M., Shams, J., Sahraei, H., Hedayati, M., Asefi, F. (2013). Journal of Behavioral and Brain Science, 3, 315-319.
Ferguson, JM. (2001). Journal of Clinical Psychiatry, 3, 22-27.
Psichiatria Sociale, 14, 2.
Hamilton, M. (1960). A rating scale for depression. Journal of Neurology, Neurosurgery and Psychiatry, 23, 56-62.
Hosseinzadeh, H., Karimi, G., Niapoor, M. (2004). Acta Horticulturae, 650, 435-45.
International Standard, Saffron specification, ISO-3632-1980 (E). International Organization for Standardization, Geneva.
nternational Standard, Saffron specification, ISO 3632-1 and ISO 3632-2: 1993 (E). International Organization for Standardization, Geneva, 1993.
Karimi, GHR. , Hosseinzadeh, H., Khalegh Pana, P. (2001). Iranian journal of basic medical sciences, 4, 11-15.
Kashani, L, Raisi, F., Saroukhani, S., Sohrabi, H., Modabbernia, A., Nasehi, AA., Jamshidi, A., Ashrafi, M., Mansouri, P., Ghaeli, P., Akhondzadeh , S. (2013). Human Psychopharmacology Clinical and Experimental Journal, 28, 54-60.
Lopresti, AL. and Drummond, PD. (2014). Human Psychopharmacology: Clinical and Experimental, 29, 517-527.
Moshiri, E., Bastí, AA., Noorbala, AA., Jamshidi, AH., Abbasi, SH., Akhondzadeh, S. (2006). Phytomedicine, 13, 607-61 1.
Moussavi, S., Chatterji, S., Greens, E., Tandon, A., Patel, V., Ustun, B. (2007). Lancet, 370, 851-858.
Radloff, L.S. (1977) Applied Psychological Measurement 1: 385-401.
Shahmansouri, N., Farokhnia, M., Abbasi, SH., Kassaian, SE., Noorbala, SA., Gougol, A., Yekehtaz, H., Forghani, S., Mahmoodian, M., Saroukhani, S., Arjmandi-Beglar, A., Akhondzadeh, S. (2014). Journal of Affective Disorders, 155, 216-222.
Serretti, A., Kato, M., Ronchi, D., Kinoshita, T. (2007). Molecular Psychiatry, 12, 247-257.
Siddiqui, M., Saleh M, Basharuddin, Zamri, S. Najib M., Ibrahim, M., Noor, N., Mazha, H., Hassan, N., and Khatib A., J Pharm Bioallied Sci. 2018 Oct-Dec; 10(4): 173-180.
Ulbricht, C, Conquer, J., Costa, D., Hollands, W., lannuzzi, C, Isaac, R., Jordan, JK., Ledesma, N., Ostroff, C, Serrano, JMG., Shafferh, MD., Varghese, M. (201 1). Journal of Dietary Supplements, 8, 58-114.
Vanderkooy, JD., Kennedy, SH., Bagby, RM. (2002). Canadian Journal of Psychiatry, Revue Canadienne de Psychiatrie, 47, 174-180.

## Claims

1. A composition for use in the method for the treatment and/or prevention of a depressive disorder in conjunction with coprostatis, comprising as the active ingredients a combination of therapeutically effective amounts of an extract of saffron stigmas (*Crocus sativus L*.) (1) and non-pathogenic probiotic microorganisms (2) and an acceptable excipient, wherein the ratio of (1) to (2) ranges from 100 to 1 to 1 to 100 by weight, **characterized in that** (2) comprises *Bifidobakterium bifidum, Bifidobakterium breve, Bifidobakterium longum, Lactobacillus acidophilus, Lactobacillus casei, Lactobacillus reuteri, Lactobacillus rhamnosus, Lactobacillus plantarum, Lactococcus lactis, and Enterococcus faecium.*

2. A composition for use according to claim 1, wherein the depressive disorder is a treatment-resistant depressive disorder.

3. A composition for use according to claim 1 or 2, wherein the administration thereof is oral.

4. A composition for use according to any one of claims 1 to 3, which is in the form of tablets, hard shell capsules or soft gel capsules.

5. A composition in the form of a tablet or hard shell capsule for use according to any one of claims 1 to 4 which further comprises one or more excipients selected from the group consisting of binders, disintegrants, fillers, glidants, and preservatives.

6. A composition in the form of a soft gel capsule for use according to any one of claims 1 to 4, which further comprises one or more excipients selected from the group consisting of gelatin, polymers based on starch or carrageenan, opacifiers, plasticizers and water.

7. A composition for use according to any one of claims 1 to 6, wherein the ratio of the extract of saffron stigmas (1) to the non-pathogenic probiotic microorganisms (2) ranges from 20 to 1 to 1 to 20 by weight.

8. A composition for use according to any one of claims 1 to 7 comprising
• 10 to 150 mg of an extract of saffron stigmas (1), and
• 1 x 10 cfu to 1 x 10¹¹ cfu of the non-pathogenic probiotic microorganisms (2),
wherein the ratio of (1) to (2) ranges from 100 to 1 to 1 to 100 by weight.

9. A composition for use according to any one of claims 1 to 8, wherein the effective amounts
• of an extract of saffron stigmas (1) is from about 10 mg to about 150 mg per day, and
• of the non-pathogenic probiotic microorganisms (2) is from about 1 x 10 cfu to about 1 x 10¹¹ cfu per day,
wherein the ratio of (1) to (2) ranges from 100 to 1 to 1 to 100 by weight.

10. A composition for use according to any one of claims 1 to 9, wherein the depressive disorder is reduced by at least 20 % according to the Center for Epidemiologic Studies Depression (CES-D) Scale.

11. A composition for use according to any one of claims 1 to 10, wherein the composition is prepared from a ready-to-use kit of parts essentially consisting of
(A) a first compartment containing a pharmaceutical composition comprising effective amounts of an extract of saffron stigmas (*Crocus sativus L*.) (1) and an acceptable excipient;
(B) a second compartment containing a pharmaceutical composition comprising non-pathogenic probiotic microorganisms (2) and an acceptable excipient;
(C) optionally a leaflet describing the dosage and administration of each of the pharmaceutical compositions (A) and (B),
wherein the ratio of (1) to (2) ranges from 100 to 1 to 1 to 100 by weight, **characterized in that** (2) comprises *Bifidobakterium bifidum, Bifidobakterium breve, Bifidobakterium longum, Lactobacillus acidophilus, Lactobacillus casei, Lactobacillus reuteri, Lactobacillus rhamnosus, Lactobacillus plantarum, Lactococcus lactis, and Enterococcus faecium.*

## Patentansprüche

1. Zusammensetzung zur Verwendung in dem Verfahren zur Behandlung und/oder Vorbeugung einer depressiven Störung in Verbindung mit Koprostase, umfassend als Wirkstoffe eine Kombination aus einer therapeutisch wirksamen Menge eines Extrakts von Safran-Narben (*Crocus sativus L*.) (1) und nichtpathogenen probiotischen Mikroorganismen (2) und einen verträglichen Hilfsstoff, wobei das Verhältnis von (1) zu (2) im Bereich von 100 zu 1 bis 1 zu 100 nach Gewicht liegt, **dadurch gekennzeichnet, dass** (2) *Bifidobacterium bifidum, Bifidobacterium breve, Bifidobacterium longum, Lactobacillus acidophilus, Lactobacillus casei, Lactobacillus reuteri, Lactobacillus rhamnosus, Lactobacillus plantarum, Lactococcus lactis* und *Enterococcus faecium* umfasst.

2. Zusammensetzung zur Verwendung nach Anspruch 1, wobei die depressive Störung eine behandlungsresistente depressive Störung ist.

3. Zusammensetzung zur Verwendung nach Anspruch 1 oder 2, wobei die Verabreichung davon oral erfolgt.

4. Zusammensetzung zur Verwendung nach einem der Ansprüche 1 bis 3, die in Form von Tabletten, Hartschalenkapseln oder Weichgelkapseln vorliegt.

5. Zusammensetzung in Form einer Tablette oder Hartschalenkapsel zur Verwendung nach einem der Ansprüche 1 bis 4, die ferner einen oder mehrere Hilfsstoffe umfasst, ausgewählt aus der Gruppe, bestehend aus Bindemitteln, Sprengmitteln, Füllstoffen, Fließregulierungsmitteln und Konservierungsmitteln.

6. Zusammensetzung in Form einer Weichgelkapsel zur Verwendung nach einem der Ansprüche 1 bis 4, die ferner einen oder mehrere Hilfsstoffe umfasst, ausgewählt aus der Gruppe, bestehend aus Gelatine, Polymeren auf Basis von Stärke oder Carrageen, Trübungsmitteln, Weichmachern und Wasser.

7. Zusammensetzung zur Verwendung nach einem der Ansprüche 1 bis 6, wobei das Verhältnis des Extrakts aus Safran-Narben (1) zu den nichtpathogenen probiotischen Mikroorganismen (2) im Bereich von 20 bis 1 bis 1 bis 20 nach Gewicht liegt.

8. Zusammensetzung zur Verwendung nach einem der Ansprüche 1 bis 7, umfassend
• 10 bis 150 mg eines Extrakts aus Safran-Narben (1) und
• 1 x 10 KBE bis 1 x 10¹¹ KBE der nichtpathogenen probiotischen Mikroorganismen (2),
wobei das Verhältnis von (1) zu (2) im Bereich von 100 zu 1 bis 1 zu 100 nach Gewicht liegt.

9. Zusammensetzung zur Verwendung nach einem der Ansprüche 1 bis 8, wobei die wirksame Menge
• eines Extrakts aus Safran-Narben (1) von etwa 10 mg bis etwa 150 mg pro Tag beträgt und
• der nichtpathogenen probiotischen Mikroorganismen (2) von etwa 1 x 10 KBE bis etwa 1 x 10¹¹ KBE pro Tag beträgt,
wobei das Verhältnis von (1) zu (2) im Bereich von 100 zu 1 bis 1 zu 100 nach Gewicht liegt.

10. Zusammensetzung zur Verwendung nach einem der Ansprüche 1 bis 9, wobei die depressive Störung gemäß der Depressionsskala des Center for Epidemiologic Studies (CES-D) um mindestens 20 % reduziert wird.

11. Zusammensetzung zur Verwendung nach einem der Ansprüche 1 bis 10, wobei die Zusammensetzung aus einem gebrauchsfertigen Kit von Teilen hergestellt ist, im Wesentlichen bestehend aus
(A) einem ersten Kompartiment, das eine pharmazeutische Zusammensetzung enthält, die wirksame Mengen eines Extrakts von Safran-Narben (*Crocus sativus L*.) (1) und einen verträglichen Hilfsstoff umfasst;
(B) einem zweiten Kompartiment, das eine pharmazeutische Zusammensetzung enthält, die nichtpathogene probiotische Mikroorganismen (2) und einen verträglichen Hilfsstoff umfasst;
(C) gegebenenfalls eine Packungsbeilage, die die Dosierung und Verabreichung jeder der pharmazeutischen Zusammensetzungen (A) und (B) beschreibt,
wobei das Verhältnis von (1) zu (2) im Bereich von 100 zu 1 bis 1 zu 100 nach Gewicht liegt, **dadurch gekennzeichnet, dass** (2) *Bifidobacterium bifidum, Bifidobacterium breve, Bifidobacterium longum, Lactobacillus acidophilus, Lactobacillus casei, Lactobacillus reuteri, Lactobacillus rhamnosus, Lactobacillus plantarum, Lactococcus lactis* und *Enterococcus faecium* umfasst.

## Revendications

1. Composition pour une utilisation dans le procédé de traitement et/ou de prévention d'un trouble dépressif en association avec la coprostatite, comprenant comme ingrédients actifs une combinaison de quantités thérapeutiquement efficaces d'un extrait de stigmates de safran (*Crocus sativus L*.) (1) et de micro-organismes probiotiques non pathogènes (2) et d'un excipient acceptable, dans laquelle le rapport de (1) à (2) varie de 100 à 1 à 1 à 100 en poids, **caractérisée en ce que** (2) comprend *Bifidobakterium bifidum, Bifidobakterium breve, Bifidobakterium longum, Lactobacillus acidophilus, Lactobacillus casei, Lactobacillus reuteri, Lactobacillus rhamnosus, Lactobacillus plantarum, Lactococcus lactis et Enterococcus faecium.*

2. Composition pour une utilisation selon la revendication 1, dans laquelle le trouble dépressif est un trouble dépressif résistant au traitement.

3. Composition pour une utilisation selon la revendication 1 ou 2, dans laquelle l'administration de celle-ci est orale.

4. Composition pour une utilisation selon l'une quelconque des revendications 1 à 3, qui se présente sous la forme de comprimés, de capsules à coque dure ou de capsules à gel mou.

5. Composition sous la forme d'un comprimé ou d'une capsule à coque dure pour une utilisation selon l'une quelconque des revendications 1 à 4, qui comprend également un ou plusieurs excipients choisis dans le groupe constitué par des liants, des désintégrants, des charges, des agents de glissement et des conservateurs.

6. Composition sous la forme d'une capsule à gel mou pour une utilisation selon l'une quelconque des revendications 1 à 4, qui comprend également un ou plusieurs excipients choisis dans le groupe constitué par la gélatine, des polymères à base d'amidon ou de carraghénane, des opacifiants, des plastifiants et l'eau.

7. Composition pour une utilisation selon l'une quelconque des revendications 1 à 6, dans laquelle le rapport de l'extrait de stigmates de safran (1) aux micro-organismes probiotiques non pathogènes (2) varie de 20 à 1 à 1 à 20 en poids.

8. Composition pour une utilisation selon l'une quelconque des revendications 1 et 7, comprenant
• 10 à 150 mg d'un extrait de stigmates de safran (1), et
• 1 x 10 ufc à 1 x 10¹¹ ufc de micro-organismes probiotiques non pathogènes (2),
dans laquelle le rapport de (1) à (2) varie de 100 à 1 à 1 à 100 en poids.

9. Composition pour une utilisation selon l'une quelconque des revendications 1 à 8, dans laquelle les quantités efficaces
• d'un extrait de stigmates de safran (1) sont d'environ 10 mg à environ 150 mg par jour, et
• des micro-organismes probiotiques non pathogènes (2) sont d'environ 1 x 10 ufc à 1 x 10¹¹ ufc par jour,
dans laquelle le rapport de (1) à (2) varie de 100 à 1 à 1 à 100 en poids.

10. Composition pour une utilisation selon l'une quelconque des revendications 1 à 9, dans laquelle le trouble dépressif est réduit d'au moins 20 % selon l'échelle de dépression du Center for Epidemiologic Studies (CES-D).

11. Composition pour une utilisation selon l'une quelconque des revendications 1 à 10, dans laquelle la composition est préparée à partir d'un kit prêt à l'emploi de pièces constituées essentiellement de
(A) un premier compartiment contenant une composition pharmaceutique comprenant des quantités efficaces d'un extrait de stigmates de safran (*Crocus sativus L*.) (1) et un excipient acceptable ;
(B) un second compartiment contenant une composition pharmaceutique comprenant des micro-organismes probiotiques non pathogènes (2) et un excipient acceptable ;
(C) éventuellement une notice décrivant la posologie et l'administration de chacune des compositions pharmaceutiques (A) et (B),
dans laquelle le rapport de (1) à (2) varie de 100 à 1 à 1 à 100 en poids, **caractérisée en ce que** (2) comprend *Bifidobakterium bifidum, Bifidobakterium breve, Bifidobakterium longum, Lactobacillus acidophilus, Lactobacillus casei, Lactobacillus reuteri, Lactobacillus rhamnosus, Lactobacillus plantarum, Lactococcus lactis et Enterococcus faecium.*
